# EUROPEAN PATENT APPLICATION

(11) **EP 3 009 845 A1**
(43) Date of publication of application: **20.04.2016**
(21) Application number: 14382404.3
(22) Date of filing: 17.10.2014
(51) Int. Cl.: G01N 35/00, G01N 1/38, B01F 11/00, G01N 33/53

(54) **Portable analyzer for automatically performing immunoassays, and for analyzing and interpreting the results thereof**

(71) Applicant: Laboratorios Alpha San Ignacio Pharma S.L., 50018 Zaragoza (ES)
(72) Inventor: Juez, Alejandro, 50018 Zaragoza (ES); Tobajas, Ángel, 50018 Zaragoza (ES); Enériz, Diana, 50018 Zarazoza (ES); Muñoz, José Antonio, 50018 Zaragoza (ES); Torrejón, Alberto Héctor, 50018 Zaragoza (ES); Fernández, Luis J., 50018 Zaragoza (ES); Prehn, Ricard, 50018 Zaragoza (ES); Roncalés, Miguel, 50018 Zaragoza (ES); Malo, Laura, 50018 Zaragoza (ES); Belacortu, Yaiza, 50018 Zaragoza (ES); Olivé, María del Mar, 50018 Zaragoza (ES)
(74) Representative: Hoffmann Eitle

(57) **Abstract**

The present invention provides an Analyzer adapted to receive an immunoassay cartridge comprising an imaging module including a two-dimensional light sensor and a plurality of light sources. The light sources and the two-dimensional light sensor are arranged such that the light sources are adapted to illuminate a result display section and a graphical code of the immunoassay cartridge and the two-dimensional light sensor is adapted to acquire images of both the graphical code and the result display section. The analyzer comprises an oscillator portion being adapted to cause the immunoassay cartridge to perform oscillations at a pre-set oscillation frequency. The analyzer comprises a processing section adapted to process an image of the graphical code acquired by the two-dimensional sensor to determine the pre-set oscillation frequency. The analyzer comprises a controller adapted to control the oscillator portion to cause the immunoassay cartridge to oscillate at the pre-set oscillation frequency.

## Description

### 1. Field of the invention

The present invention relates to an analyzer which is adapted to be used in combination with a corresponding immunoassay cartridge for automatically carrying out immunoassays, in particular lateral flow immunoassays, and for automatically analyzing the results of the same.

### 2. Technical background

Immunoassays are biochemical tests through which the presence and/or concentration of a substance - usually referred to as the analyte - in a solution can be determined. The determination is based on the interaction of the analyte with a corresponding substance such as an antibody or immunoglobulin. In the field of immunoassays, lateral flow immunoassays are known which usually make use of relatively cheap cartridges housing lateral flow strips for performing the lateral flow immunoassay. The lateral flow assays are immunochromatographic assays or strip tests which use pieces of porous paper or sintered polymer where the reagents (antibodies or antigens) are dry. The sample with or without the antigens, runs thought the strip thereby dragging the reagents (antibody pairs) deposited in a wick pad of the strips. When the mixture of sample and reagents reaches a test line, they can interact with the dry regents in the display section of the strip and develop or not a test line. The developing or not of a test line can be interpreted as presence or absence of the antigen in the sample (depending on the type of immunotest). Lateral flow strips can be used in home pregnancy tests. Lateral flow strips are also known in the field of sports anti-doping analysis where blood samples are tested for prohibited doping substances. A further application of lateral flow strips is the detection of drugs in drug tests carried out by the police.

In particular in view of their simplicity and their relatively cheap availability, lateral flow assay devices have been subject to numerous studies and this growing field is subject to a large number of developments. Within this growing field, a considerable number of developments aim in particular at providing electronic reading devices for reading the results of lateral flow immunoassays.

A typical example of such an optical reader system is described in document WO 2012/012500 A1. Therein, a reader is disclosed comprising a cartridge receiving member, an excitation member and a corresponding imaging system. The cartridge receiving member receives a cartridge comprising a lateral flow strip with a sample to be analyzed received thereon. The excitation member is used to illuminate an area of the cartridge and the imaging system captures an image of this area, the image being analyzed for obtaining results.

A further development in this growing field is described in document WO 2013/096804 A2 which discloses an optical assay device with a pneumatic sample actuation. This document discloses a testing and reader system for receiving a lateral flow strip and a test sample. According to this document, a sample is moved towards the lateral flow strip making use of a pneumatic pump. Optical signals are read using a dedicated imaging system which can be used to simultaneously detect an optical signal of the test strip and of a graphical code provided on the testing device. The graphical code can be used e.g. for determining information regarding the patient or the particular analyte under consideration.

The present invention is similarly set up in this growing field and in particular addresses needs arising in the field of the detection of drugs detected in saliva, urine, blood or plasma. In particular in this field, it is desirable to provide a fully automatized device capable not only of reading and analyzing test results in an improved manner but at the same time to also perform the mechanical steps of the assays by non-scientific personnel. This is important in the case of tests which need multiple steps in a particular order, such as homogenization of reagents included in a cartridge and/or mixing the sample with buffers. Further, it is desirable that such a fully automatized system can be incorporated within a small portable device which can easily be transported e.g. within a police car to locations where drug tests are to be carried out. It is necessary to allow performing and interpreting an assay correctly and reproducibly by non-scientific personnel in any place and under any condition. It is also important to perform the test in an optimum way and to include also lot-specific parameters.

It is thus an object of the present invention to improve the state of the art by providing an analyzer which - preferably in combination with a suitable immunoassay cartridge - is capable of automatically performing an immunoassay and of analyzing and interpreting the results of the same. It is further an object of the present invention to provide an analyzer which incorporates all necessary components within a small and portable device.

These and other objects which will become apparent upon reading the following description are solved by an analyzer according to claim 1, a method according to claim 13 and by an immunoassay analysis system according to claim 16.

### 3. Summary of the invention

According to the invention, an analyzer is provided which is adapted to interact with a corresponding immunoassay cartridge for automatically performing an immunoassay and for automatically analyzing and interpreting results of the immunoassay. The analyzer is adapted to at least partially receive the immunoassay cartridge. As the person skilled in the art will understand, depending on constructional details, it may be sufficient to e.g. only partially insert the immunoassay cartridge into the analyzer or the immunoassay cartridge can be fully inserted into a corresponding portion of the analyzer. In a preferred embodiment, the immunoassay cartridge is fully inserted into the analyzer which has the advantage that during the immunoassay, the cartridge is protected by the analyzer. Preferably, the immunoassay cartridge comprises a strip for performing an immunoassay such as a lateral flow immunoassay.

Preferably, the immunoassay cartridge comprises a result display section and a graphical code which is arranged on an outer surface of the immunoassay cartridge. Preferably, the immunoassay cartridge comprises one or more immunoassay strips.

The graphical code may be a two-dimensional code such as a barcode or a matrix barcode as for example a QR code or the like. "QR-code" is the abbreviated form of Quick Response code. Matrix barcodes are particularly advantageous in that with their help a large amount of information can be encrypted in a very compact way. In a preferred embodiment, the graphical code is a two-dimensional code, preferably a matrix code, most preferably a QR code.

Further, the cartridge may comprise a holder and a cassette for housing at least one immunoassay strip. The holder may be dedicatedly fabricated to interact with the analyzer for automatically performing the immunoassay, while the cassette including the strip may be a commercially available item. Thus, in a preferred embodiment, the present invention provides an immunoassay analysis system comprising the analyzer and a corresponding immunoassay cartridge, whereby the immunoassay cartridge comprises a holder and a cassette for housing an immunoassay strip.

Preferably, the result display section is an opening in an outer surface of the immunoassay cartridge, which preferably incorporates an optically transparent material such as an optically transparent plastic material. In a preferred embodiment, the result display section is an opening in an outer surface of the immunoassay cartridge facing the two-dimensional sensor. The immunoassay cartridge can in a preferred embodiment be a cartridge as developed by the American Bio Medica Corporation (ABMC) which is subject to the US Patent Application No. 14/205,631, the content of which is hereby fully incorporated herein by reference.

Further, according to the invention, the analyzer comprises an imaging module including a two-dimensional light sensor at least partially surrounded by a plurality of light sources. Preferably, the two-dimensional light sensor is a CMOS sensor or a CCD sensor. Further, preferably, the light sources are light emitting diodes, preferably white light emitting diodes. The diodes can for example be arranged within a plane of the two-dimensional sensor forming a circular or rectangular arrangement around the sensor. Such arrangement of diodes allows that a surface which is imaged by the sensor can be illuminated in an advantageous manner.

According to the invention, the light sources and the two-dimensional light sensor are arranged such that when the immunoassay cartridge is at least partially received by the analyzer, the light sources are adapted to illuminate at least the result display section and the graphical code of the immunoassay cartridge. Further, the two-dimensional light sensor is adapted to acquire images of both the graphical code and the result display section. In other words, the graphical code and the result display section can be for example on the same side of the cartridge which faces the two-dimensional light sensor when the cartridge is received by the analyzer or inserted into the analyzer. Further, the result display section can for example be a section of the cartridge through which the results of the immunoassay, e.g. corresponding result lines are visible.

According to the invention, the analyzer further comprises a mechanical module comprising a plurality of actuators and an oscillator portion. Thereby, each of the actuators is adapted to interact with a corresponding portion of the immunoassay cartridge, when the immunoassay cartridge is at least partially received by the analyzer for automatically performing an immunoassay. Preferably, the analyzer comprises three actuators which are linear actuators movable essentially in linear directions towards the immunoassay cartridge when the immunoassay cartridge is at least partially received by the analyzer. In other words, the analyzer is provided with a mechanical module which allows that the analyzer automatically performs steps of performing an immunoassay with the immunoassay cartridge which otherwise would have to be performed by an operator. The actuators interact with corresponding portions of the cartridge, e.g. are adapted to press a corresponding portion into the cartridge or the like while by means of the oscillator portion the cartridge can be set into oscillation e.g. in order to appropriately produce a mixture.

Further, according to the invention, the oscillator portion is arranged such that when the immunoassay cartridge is received by the analyzer, the oscillator portion is in mechanical contact with the immunoassay cartridge, the oscillator portion thereby being adapted to cause the immunoassay cartridge to perform oscillations at a pre-set oscillation frequency. As it will be known to the person skilled in the art, upon performing immunoassays using immunoassay cartridges e.g. upon performing drug tests, it is usually necessary to oscillate the cartridge, e.g. to manually shake the cartridge, for one or more dedicated times. However, such manual shaking may not be always performed for a sufficient time or not at a frequency optimal for the immunoassay. In contrast, the inventive device allows to oscillate, i.e. to shake, the immunoassay cartridge at a pre-set frequency in a controlled manner, e.g. for a controlled time.

In a preferred embodiment, the oscillator portion comprises an oscillator plate and the analyzer and the immunoassay cartridge are provided such that when the immunoassay cartridge is received by the analyzer, the immunoassay cartridge is placed on the oscillator plate. Thereby, the oscillator portion can be an oscillator plate connected to a corresponding motor for causing the plate to oscillate at the pre-set frequency. When the immunoassay cartridge is for example inserted into the analyzer, the immunoassay cartridge is then placed on the oscillator plate such that upon actuation of the corresponding motor, the oscillator plate and accordingly the immunoassay cartridge are oscillated, i.e. shaken, at the oscillation frequency determined by the motor.

The analyzer according to the invention further comprises a processing section adapted to process an image of the graphical code acquired by the two-dimensional sensor to determine the pre-set oscillation frequency. In other words, the graphical code which in a preferred embodiment is a two-dimensional code, preferably a matrix barcode, is used to encrypt information on the particular immunoassay cartridge it is placed on. For example, the graphical code can include information on parameters important for performing the immunoassay such as the optimal oscillation frequency and for analyzing and interpreting the same.

Thus, according to the invention, the analyzer further comprises a controller connected to the processing section and to the oscillator portion, the controller being adapted to receive, from the processing section, the pre-set oscillation frequency. The controller according to the present invention is further adapted to control the oscillator portion to cause the immunoassay cartridge to oscillate at the pre-set oscillation frequency.

In other words, the graphical code includes information on an oscillation frequency which is an oscillation frequency ideal for example to homogenize a dilution comprising a substance for analysis of a sample subject to the immunoassay and a sample. This oscillation frequency can thus be encrypted into the graphical code and automatically read by the two-dimensional sensor. It becomes thus possible that even if the analyzer is used by untrained personnel, an oscillation of the immunoassay cartridge is automatically set which is ideal for the sample and a dilution used therein. As opposed to manual shaking, with the inventive device it becomes thus possible that in an automated fashion, the immunoassay cartridge can be oscillated at the ideal oscillation frequency.

The present inventors determined experimentally that preferably, said oscillation frequency is in between 0,1 Hz and 100 Hz, preferably in between 0,5 Hz and 10 Hz, more preferably in between 1 Hz and 5 Hz, and most preferably in between 2 Hz and 3 Hz, when the sample to be analyzed by the immunoassay is a saliva sample or urine.

In a preferred embodiment, the oscillator portion comprises a position detection sensor adapted to detect a position of the immunoassay cartridge with respect to the analyzer, when the immunoassay cartridge is received by the analyzer. This sensor can for example be an optical sensor or an electronic sensor which preferably is connected to the controller. The provision of a position detection sensor at the oscillator portion has been found to be particularly advantageous when for example an immunoassay cartridge is inserted into the analyzer by untrained personnel moving the oscillator portion in an undesirable way. By providing the position detection sensor at the oscillator portion, it can be assured that the sensor is always capable of detecting the position of the immunoassay cartridge with respect to the analyzer. In a preferred embodiment, the controller is adapted to position the immunoassay cartridge with respect to the analyzer in response to a signal of the position detection sensor. For example, the oscillations of the oscillator portion can be caused by a turning wheel to which the oscillator portion is eccentrically connected. The wheel can be provided with a mark which can be monitored by the position detection sensor. Using this setup, the cartridge can be set to a position by positioning the mark of the wheel which is monitored by the position detection sensor. It can thus be assured that the immunoassay cartridge is correctly placed and the immunoassay can be performed as desired. Thus, in a preferred embodiment, the oscillator portion is eccentrically connected to a wheel, such that by turning the wheel, oscillations of the oscillator portion are generated. Preferably, the wheel is provided with a mark and the position detection sensor is provided such that it can detect the mark, the analyzer thereby being adapted to position the oscillator portion at a predetermined position. Preferably, the position detection sensor is an optical sensor. It turned out that an optical sensor is in particular advantageous since it has a particularly low drift of position measurements over time as compared to other types of sensors.

In a preferred embodiment, the plurality of actuators further comprises at least a first actuator, a second actuator and a third actuator, each being arranged moveable such that when the immunoassay cartridge is received by the analyzer, each of the actuators is adapted to be moved from an initial position in a direction towards the immunoassay cartridge. Preferably, upon moving in this way, each of the actuators is adapted to engage a corresponding portion of the immunoassay cartridge and thus upon further movement for example to move a corresponding part of the immunoassay cartridge.

Thereby, the controller is adapted to automatically control movement of each of the actuators. In other words, the analyzer may comprise storage means for storing dedicated software which includes instructions for the controller how to control each of the actuators in order to automatically perform the immunoassay. Thus, after starting the analyzer e.g. by pressing a corresponding button, an operator does not have to interact with the analyzer, but the analyzer can interact with the inserted cartridge for automatically performing the immunoassay through the use of the actuators which are automatically controlled by the controller.

In a preferred embodiment, the controller controls movement of each of the actuators in accordance with information received from the processing section and determined from processing an image of the graphical code acquired by the two-dimensional light sensor.

In a preferred embodiment, each actuator can be an essentially linearly shaped member, e.g. a linearly shaped mechanical tool connected to a corresponding motor arranged on the analyzer such that said member or said tool can be moved towards an inserted immunoassay cartridge to interact with the cartridge. Thereby, the term "essentially" reflects that the actuator can be a member which is mainly linearly shaped, i.e. is shaped along a linear axis. As it will be clear for the skilled person a corresponding essentially linearly shaped tool may have deviations from a linear shape e.g. as they are necessary for forming the tool.

Preferably, each actuator is provided with an electrical motor connected to a corresponding spindle which in turn is mechanically connected to the linear actuator such that a rotatory movement of the motor is converted into a linear movement of the actuator. It was found that such a configuration is particularly advantageous in that the rotatory movement of the motor can be used to move the actuator in a particularly controlled manner and in that forces necessary for the corresponding interaction with the cartridge can be transferred in a particularly controlled manner.

Further, preferably, the first actuator, the second actuator and the third actuator are linearly moveable in a direction essentially perpendicular to the oscillator plate. Herein, terms such as "essentially" perpendicular or "essentially" parallel reflect the usual tolerances as they may occur due to production imperfections or the like. Thus, "essentially perpendicular" or "essentially parallel" include angular deviations from a perfectly linear or parallel in a mathematical sense within a range of 0° to 10°, preferably 0° to 7.5°, more preferably 0° to 5°, and most preferably 0° to 2.5°.

It was found that the above described construction of the actuators moving in the described fashion towards the oscillator plate is advantageous in that thus the actuators can optimally interact with a correctly inserted cartridge. The cartridge in turn can be optimally supported using the above described oscillator plate. This arrangement further allows that for example, a force applied by one of the actuators to a corresponding portion of the cartridge can be optimally transferred onto the cartridge while using the optical position detection sensor preferably provided within the oscillator plate it can be assured that the immunoassay cartridge is appropriately and correctly placed. Thus, using the position detection sensor, a correct function of the actuators can be advantageously assured by assuring that the cartridge is correctly positioned on the oscillator portion, preferably the oscillator plate.

Further, in a preferred embodiment, the analyzer comprises at least one actuator position sensor for a corresponding one of the actuators. In a preferred embodiment, the actuator position sensor is a potentiometer. It turned out that this type of sensor is beneficial as compared to other sensors since it allows for a particularly high repeatability which attributes to an enhanced lifetime of the whole system. The actuator position sensor is connected to the controller and is adapted to convert a position of the corresponding actuator into a signal, preferably a voltage signal. Preferably, the controller is in turn adapted to control the position of the corresponding actuator by controlling a corresponding motor which is mechanically connected to the corresponding actuator in response to the voltage signal.

For example, each actuator can be provided with its own motor whereby for example a rotating action of the motor is converted into a linear movement of an actuator making use of a dedicated spindle or shaft. Thus, preferably the analyzer comprises four motors controlled by the controller, three for each actuator being adapted to linearly move each actuator and one motor for causing the oscillator portion to oscillate.

In a preferred embodiment, at least one of the actuators is adapted to compress a corresponding portion of the immunoassay cartridge upon engagement with the corresponding portion of the immunoassay cartridge. Preferably, this actuator is adapted to perforate said corresponding portion of the immunoassay cartridge upon engagement with the corresponding portion of the immunoassay cartridge. In other words, upon movement from the initial position towards the immunoassay cartridge, a portion of the actuator may engage a corresponding portion and cause a compression of the same. Thereby, e.g. a substance within the cartridge may be pressed and thus moved from one portion of the cartridge to another portion.

Further, a portion of the actuator which may be a sharpened point may upon engagement with said corresponding portion of the cartridge and upon further movement of the actuator, perforate the corresponding portion thus producing a hole. This particular actuator allows that e.g. an inner chamber of the cartridge may be perforated such that a dilution is brought into contact with a substance for analyzing the analyte e.g. with antibodies provided at an inner surface of the inner chamber such that the dilution can be mixed with the antibodies. Alternatively, a mixture of dilution and antibodies can be achieved by compressing a flexible portion of the immunoassay cartridge, thus introducing a dilution comprising a substance for analysis of the sample, preferably comprising antibodies, into an inner chamber of the cartridge. Said compression can be achieved by the action of a corresponding actuator.

For example, an operator may insert the immunoassay cartridge into the analyzer and press a button to start the fully automatized immunoassay procedure which preferably is carried out using a dedicated algorithm which may be stored on a CPU connected to the controller. One of the steps carried out may cause the controller to cause the actuator which is adapted to perforate the portion of the immunoassay cartridge to be moved towards the cartridge and thus perforate a corresponding portion such that a mixture of dilution and antibodies can be achieved in a fully automated manner. Due to the dedicated algorithm, the controller can then further cause the oscillator portion to oscillate at the pre-set frequency such that an optimally homogenized mixture can be achieved.

In a preferred embodiment, at least one of the actuators, preferably two of the actuators, is, preferably are, provided with a guiding portion, and in that upon movement from the initial position towards the immunoassay cartridge, the at least one actuator is adapted to guide a sample collection member at least partially into an inner chamber of the immunoassay cartridge. Preferably, the sample collection member comprises a stick or handle and an absorbent portion, e.g. a sponge or cotton portion, adapted to absorb a sample to be subjected to the immunoassay. For example the sample collection member can be a stick with a cotton portion to be inserted into the mouth of a person to collect saliva which then is to be subjected to the immunoassay.

In a preferred embodiment, the sample collection member is pre-positioned in a first position when the immunoassay cartridge is received by the analyzer and the at least one actuator is adapted to guide the sample collection member from this first position further into the inner chamber into a second position in which position preferably the sample comes into contact with a dilution comprising a substance for analyzing the sample such as antibodies.

In a preferred embodiment, at least one of the actuators is provided with a contact portion and upon movement from the initial position towards the immunoassay cartridge, the contact portion of the at least one of the actuators is adapted to come into contact with a corresponding portion of the immunoassay cartridge to move at least one test strip at least partially into an inner chamber of the immunoassay cartridge. For example, one or more test strips can be provided within a portion of the cartridge such as housed within a cassette which upon interaction with the actuators is moved further into the cartridge such that the at least one test strip(s) preferably come(s) into contact within a mixture of the dilution and the sample, i.e. the analyte, inside of the inner chamber.

It was further found that it is advantageous to control the internal temperature of the analyzer in order to ensure optimal performance. It was found that optimum conditions are assured when the temperature is between 10°C and 40°C, more preferably in between 15°C and 39°C, even more preferably in between 18°C and 38°C and most preferably in between 20°C and 37°C.

In a preferred embodiment, the analyzer further comprises a printer, preferably a thermal printer, for automatically printing results of the immunoassay, a screen, preferably an LCD screen, for displaying results of the immunoassay and a storage for storing results of the immunoassay, and incorporates the imaging module, the oscillator portion, the processing section, the controller, the first actuator, the second actuator, the third actuator, the printer, the screen and the storage in a single portable device with a total weight of less than 10 kg, preferably of less than 7,5 kg, more preferably of less than 5 kg and most preferably of less than 2,5 kg.

Thus, the inventive construction allows to achieve a portable analyzer which preferably allows not only to read immunoassay results but also to analyze and interpret the results of the same while the device is small and portable and can be easily transported in a car such as for example a police car.

In a preferred embodiment, the analyzer is provided with a CPU connected to the controller and with storage means for storing a dedicated algorithm. The algorithm is dedicated for the analyzer for causing the controller to cause the analyzer to automatically interact with an inserted immunoassay cartridge to perform an immunoassay.

The inventive device thus allows advantageously to automatically perform an immunoassay without the necessity for scientifically trained personnel. According to the invention thus, a method is provided for automatically performing an immunoassay using the analyzer as described above and a corresponding immunoassay cartridge. Thereby, the inventive method comprises the following steps which are performed preferably in the given order:

The method comprises a step A of inserting a sample collection member comprising a sample to be analyzed by the immunoassay into a first position at least partially inside the immunoassay cartridge.

Further, the method comprises a step B of inserting the immunoassay cartridge at least partially into the analyzer.

Further, the method comprises a step D1 of controlling a first actuator to be moved from an initial position towards the immunoassay cartridge, thereby compressing a flexible portion of the immunoassay cartridge, thus introducing a dilution, preferably comprising a reagent, for analysis of the sample, preferably comprising a buffer for homogenizing reagents, preferably comprising antibodies, into an inner chamber of the immunoassay cartridge.

Further, the method comprises a step E of controlling the oscillator portion to cause the immunoassay cartridge to perform first oscillations at a pre-set oscillation frequency. For example, this first step of oscillating can serve to provide a homogeneous mixture of the dilution and a reagent provided in the dilution. Alternatively, the reagent can be a solid state reagent provided at the inner walls of an inner chamber of the immunoassay cartridge and the first oscillations may serve to dissolve the solid state reagent from the inner wall of the immunoassay cartridge. In a preferred embodiment, the immunoassay cartridge comprises a holder and a cassette including an immunoassay strip and the holder comprises an inner chamber, the inner chamber comprising inner walls, wherein the inner walls are provided with a solid state reagent. It turned out that the use of a solid state reagent is in particular advantageous if the substance to be detected is the Cannabis metabolite (tetrahydrocannabinol, THC). In this case, as solid state reagent is anti-THC-antibodies are used.

Further, the method comprises a step D2 of controlling a second actuator to be moved from an initial position towards the immunoassay cartridge into engagement with the sample collection member, thereby guiding the sample collection member including the sample to be analyzed at least partially into the inner chamber of the immunoassay cartridge into contact with the dilution to form a mixture of sample and dilution.

Further, the method comprises a step F of controlling the oscillator portion to cause the immunoassay cartridge to perform second oscillations at a pre-set oscillation frequency in order to homogenize the mixture of the dilution and the sample. In a preferred embodiment, the pre-set oscillation frequency in step F is the same pre-set oscillation frequency as the one of step E.

It turned out that optimal results can be achieved if the oscillation frequency is within a range of 0,1 to 100 Hz, preferably within a range of 0,5 to 10 Hz, more preferably within a range of 1 to 5 Hz, and most preferably within a range of 2 to 3 Hz, in particular when the sample to be analyzed by the immunoassay is a saliva or urine sample.

Further, the method comprises a step G of controlling a third actuator to be moved from an initial position towards the immunoassay cartridge, thereby moving at least one test strip at least partially into the inner chamber of the immunoassay cartridge into contact with the mixture. Depending on the immunoassay, it may be sufficient to use a single test strip. However, also more than one test strip may be moved into said inner chamber if necessary.

Further, the method comprises a step H of waiting for a pre-set time to allow incubation, i.e. contact, of the test strip with the mixture. In a preferred embodiment, the method further comprises determining the pre-set time via image processing of a graphical code provided at the immunoassay cartridge 200.

It turned out that optimal results can be achieved if the pre-set time is within a range of 15 s to 9 min, preferably within a range of 2 min to 8 min, more preferably within a range of 3 min to 7 min, and most preferably within a range of 4 min to 6 min.

Further, the method comprises a step I of acquiring a result image of a result display section of the immunoassay cartridge.

Further, the method comprises a step J of processing and analyzing the result image in order to determine as a result result-substances comprised by the sample, wherein steps D1 to J are performed automatically by the analyzer.

Thus, according to this method it is only necessary that a person inserts the immunoassay cartridge into the analyzer, start the operation of the analyzer e.g. by pressing a button of the analyzer and wait until all of the above steps have been carried out automatically. Thus, even fully untrained personnel can use the analyzer to in fully automated fashion perform an immunoassay and analyze and interpret the results of the same.

In a preferred embodiment, the method further comprises a step C1 of acquiring an image of a graphical code, preferably of a matrix barcode, provided at the immunoassay cartridge. Further, the method preferably comprises a step C2 of processing the image of the graphical code in order to determine at least one of the pre-set oscillation frequency and/or the pre-set time, wherein steps C1 and C2 are performed before step D, and wherein steps C1 to J are performed automatically by the analyzer.

Still further preferred, in step J, it is determined that a result-substance is present in the sample, when a detected signal corresponding to the result-substance is above an internal threshold. The internal threshold may be a device dependent threshold. For example, parameters of the immunoassay may change depending on the cartridge. With conventional devices such as readers of immunoassay devices, parameters often have to be newly adjusted due to lot to lot variances of immunoassay cartridges. Thus, also thresholds may vary depending on the cartridge used above which for example an immunoassay line is detected to be above the noise level. Such an internal threshold may vary for the immunoassay cartridge in question from lot to lot such that it is particularly advantageous if such information is encrypted into the graphical code provided on the cartridge. In this case, the internal threshold is set automatically and the immunoassay result can be assured to be reproducible even if different cartridges are used which are from different lots.

Further, in a preferred embodiment, when it is determined that a result-substance is present in the sample, a positive result is output when a concentration of the result-substance in the sample derived from the detected signal is above a threshold concentration. Preferably, thereby step C2 further comprises processing the image of the graphical code in order to determine the threshold concentration. A threshold concentration is e.g. a concentration above which a result of an immunoassay is judged to be positive. Such threshold concentration may be known to a user. However, it is in particular advantageous if such threshold concentration is encrypted into the graphical code, in particular if the analyzer is used by untrained personnel. In this case, such untrained personnel do not have to be informed about certain specific concentrations but can simply perform the immunoassay and get a result as being positive or negative.

The present invention provides thus an immunoassay analysis system comprising the analyzer as described above and a corresponding immunoassay cartridge. In a preferred embodiment, in the immunoassay analysis system, the immunoassay cartridge comprises a holder and a cassette for housing an immunoassay strip.

In a further preferred embodiment, an immunoassay analysis system is provided comprising the analyzer as described above and a holder for a cassette comprising an immunoassay strip. Thereby, the graphical code is provided on a surface of the holder and the graphical code encodes a pre-set internal threshold and a pre-set threshold concentration. Thereby, the analyzer is adapted to automatically analyze a result of an immunoassay and determine that a result-substance is present in a sample when a detected signal corresponding to the result-substance is above the internal threshold. Still further, thereby, the analyzer is adapted to output a positive result when a concentration of the result-substance in the sample derived from the detected signal is above the threshold concentration.

### 4. Description of the preferred embodiments

In the following, the invention is described exemplarily with reference to the enclosed figures in which:
Fig. 1 shows a schematic illustration of an analyzer 100 with a corresponding immunoassay cartridge 200 received by the analyzer;
Fig. 2 shows a schematic exploded view of the analyzer 100;
Fig. 3 shows a schematic illustration of a lateral flow strip;
Fig. 4 shows exemplarily examples of an immunoassay; and
Figs. 5 and 6 show the results of Fig. 4 as corresponding curves.

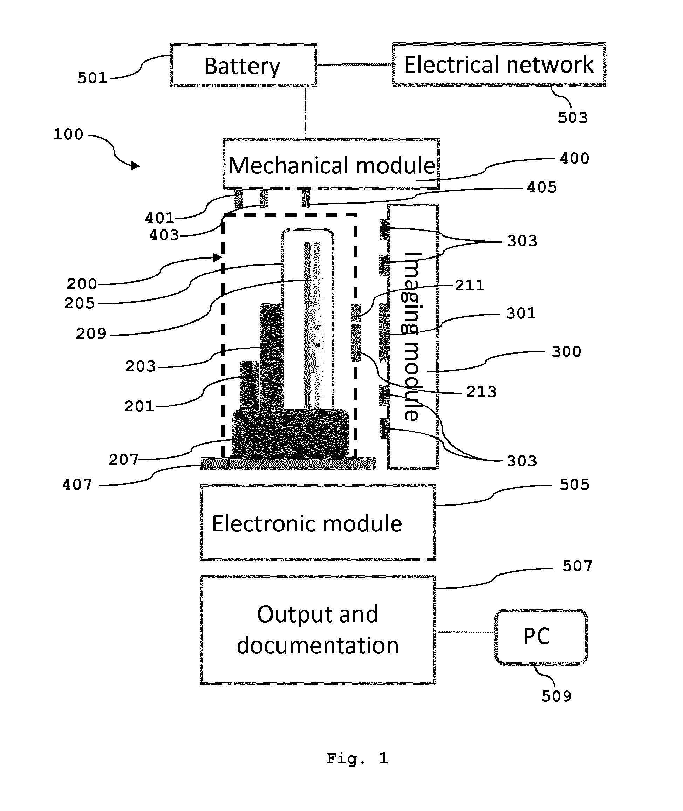

Fig. 1 shows a schematic view of an analyzer 100. As illustrated in this schematic figure, the immunoassay cartridge 200, which is schematically shown by the dashed square, is received by the analyzer 100, i.e. is placed with respect to an imaging module 300 comprising a CMOS sensor 301 (alternatively, a CCD sensor may be used) such that a result display section 213 and a graphical code 211 (both only schematically shown) face the CMOS sensor 301. The dashed line schematically illustrates a holder for housing a cassette 205 which in turn houses a lateral flow strip 209 for performing the immunoassay. As mentioned above, while the cassette may be a commercially available, standardized component, the holder preferably is dedicatedly fabricated for the analyzer thus allowing for a particularly beneficial interaction between the analyzer and the cartridge comprising the holder and the cassette.

The result display section 213 may be an opening in an outer surface or an outer shell or wall of the immunoassay cartridge 200 which faces the CMOS sensor 301. This opening can be covered by a transparent material such as a transparent plastic sheet.

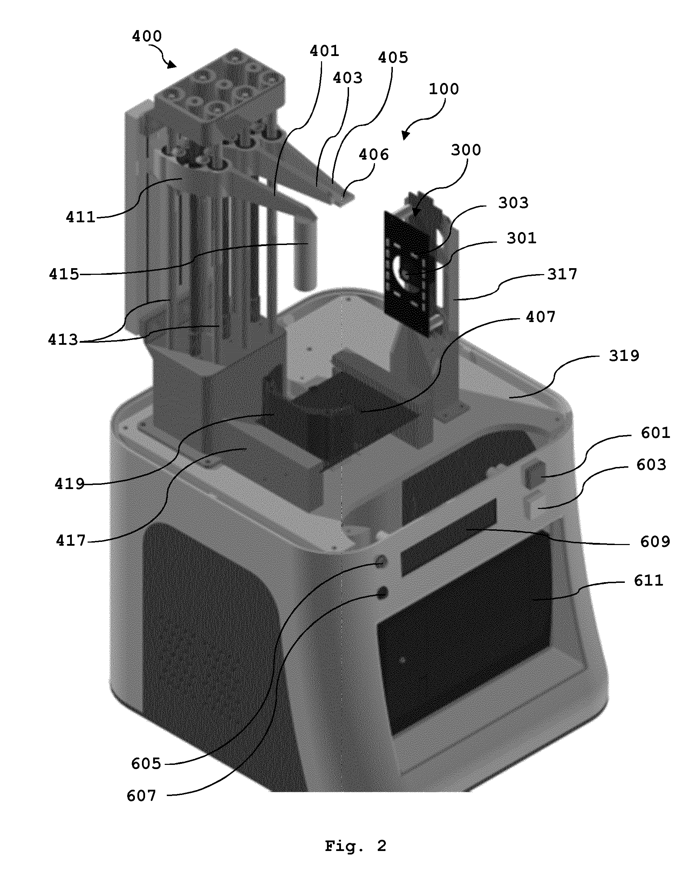

As illustrated in Fig. 1, the analyzer is provided with a mechanical module 400 which is provided with three schematically shown linear actuators 401, 403, 405. In the shown case where the immunoassay cartridge 200 is received by the analyzer 100, the immunoassay cartridge 200 is placed on an oscillator portion, e.g. an oscillator plate 407. As can be taken from this figure, the linear actuators 401, 403 and 405 are arranged movable in a direction essentially perpendicular to the orientation of the oscillator plate 407. As the skilled person will understand, upon a movement of the linear actuators 401 and 403 downwards in the figure, these actuators will engage corresponding portions 201, 203 of the immunoassay cartridge 200. For example, with reference to Fig. 2 it is shown that the actuator 405 is provided with a contact portion 406 at its end which is adapted to come into contact with a corresponding portion of the immunoassay cartridge 200 to move e.g. one or more test strips at least partially into an inner chamber of the immunoassay cartridge.

Turning back to Fig. 1, it can be seen that similarly, upon movement for example of the actuator 403 downwards in the figure, the portion 203 will be pressed and moved downwards in the figure further into an inner chamber (not visible in the figure) provided inside the portion 207. For example, the portion 203 can be a flexible portion of the immunoassay cartridge which becomes compressed due to the interaction with the linear actuator 403 whereby a dilution absorbed by the flexible portion is introduced into the inner chamber provided inside the portion 207 of the immunoassay cartridge 200.

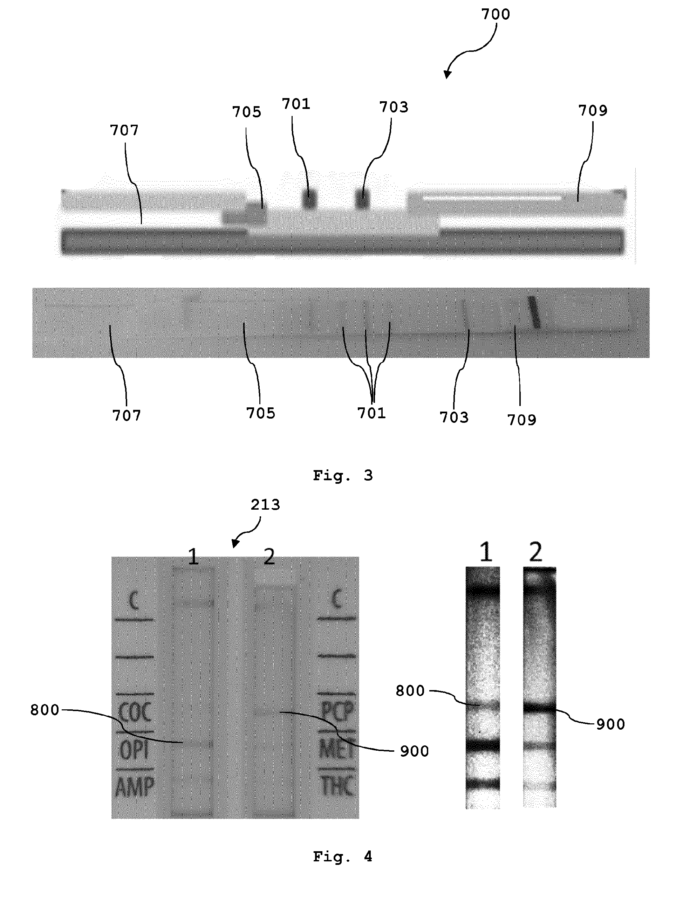

Further, Fig. 1 shows the cassette 205 which houses a lateral flow strip 209 for performing the immunoassay which is shown in more detail in Fig. 3. As shown in Fig. 1, in order to assure an optimal illumination of the lateral flow strip 209, the optical module 300 is provided with a plurality of light sources 303 which in the shown embodiment are LEDs 303. It was found that optimal performance of the analyzer can be achieved by using white light LEDs 303 surrounding a CMOS or CCD sensor 301.

As further shown, the analyzer 100 can be operated using simple batteries 501 which contribute to the light weight construction of the analyzer as compared to power supplies as they are often necessary for large laboratory devices. It was found that using a battery 501 with a capacity 6000 mAh for an LCD screen which can be incorporated with the analyzer 100 and the electronics and using a 2000 mAh battery for a printer incorporated within the analyzer, a total weight of only about 2,4 kg can be achieved.

Further, schematically shown are an electrical network 503 which is used to connect the individual components with an electronic module 505, an output and documentation portion 507 and a personal computer 509. The electronic module 505 may include a processing section adapted to process an image of the graphical code 211 acquired by the two-dimensional sensor 301 to determine the pre-set oscillation frequency. The electronic module 505 may further include a controller connected to the processing section and to the oscillator portion 207. The controller may thus be adapted to receive, from the processing section, the pre-set oscillation frequency and may be adapted to cause the immunoassay cartridge 200 to oscillate at the pre-set oscillation frequency.

For example, the CMOS sensor 301 can be connected to the electronic module 505 and adapted to read the graphical code 211 to determine a pre-set oscillation frequency. The oscillator plate 407 can be connected to the electronic module 505 and the controller provided within the electronic module 505 is adapted to control oscillation of the oscillator plate 407 at the pre-set oscillation frequency determined by the electronic module through processing of the image of the graphical code 211 acquired by the CMOS sensor 301.

The electronic module 505 may further include a CPU connected to the controller and storage means. The storage means may be used for saving an algorithm run using the CPU to cause the analyzer 100 to perform actions necessary for performing an immunoassay in connection with the immunoassay cartridge 200 in an automated manner. At the same time, the algorithm may include steps for processing result images of the immunoassay acquired using the optical module 300. Thus, the analyzer 100 according to the present invention allows for performing an immunoassay, acquiring result images of the immunoassay and processing the same in a fully automated manner making use of a small, lightweight modular device which can e.g. easily be transported in a police car.

Further, the analyzer 100 is provided with an output and documentation portion 507 including for example a thermal printer which can automatically print the results of an immunoassay. Further, the output and documentation portion 507 can include an LCD screen for displaying the results of the immunoassay and the output and documentation portion 507 can be connected to a personal computer for saving results of the immunoassay.

Fig. 2 shows a schematic exploded view of the analyzer 100. As shown in this exploded view, the linear actuators 401, 403 and 405 extend from respective base portions 411 (only one indicated in the figure) in directions essentially parallel to a plane of the oscillator plate 407. As shown, the base portions 411 travel along respective guide rails 413 when the actuators 401, 403, 405 move from the initial position as shown in Fig. 2 towards the immunoassay cartridge (not shown in the figure), i.e. downwards in Fig. 2. In a preferred embodiment, two guide rails are provided for each actuator. As may be taken from Fig. 2, by the advantageous provision of two guide rails 413 for each linear actuator a particularly controlled and stable linear movement of the linear actuators in a direction essentially perpendicular to the oscillator plate is assured.

As shown in Fig. 2, the actuator 401 is provided with a guiding portion 415 extending in a direction essentially perpendicular to the plane of the oscillator plate 407. This guiding portion 415 is adapted to receive a portion of a sample collection member (not shown in the figure) such as a stick connected to an absorbing portion for absorbing a sample for carrying out the immunoassay. As can be taken from the figure, the guiding portion preferably is an essentially cylindrically shaped member adapted to receive a linear portion, i.e. a stick-like portion, of the sample collection member. Thus, a particularly stable and controlled guidance may be achieved.

Further, Fig. 2 illustrates the optical module 300 exemplarily showing LEDs 301 (only one indicated in the figure) which surround a CMOS or CCD sensor 301 provided in the center. The optical module 300 is provided fixedly attached to a support structure 317 which extends from a supporting plate 319 in a direction essentially perpendicular to the orientation of the oscillator plate 407. As shown also in the figure, the support plate 319 also supports holding portions 417 (only one indicated in the figure) which hold the oscillator portion 407 in a manner that an oscillation is controlled to be in a plane perpendicular to the movement of the linear actuators, i.e. upon oscillation the oscillator plate 407 can slide within corresponding grooves provided within the holding portions 417. Still further, a holding member 419 is provided extending from the oscillator plate 407 such that this holding member can receive the immunoassay cartridge and support the same in a secured manner.

Fig. 2 further shows two buttons 601 and 603 for starting the action of the analyzer and two corresponding lights 605 and 607 indicating start and stop of the immunoassay. Thus, even untrained personnel can easily operate the analyzer 100 since it is only necessary to insert the immunoassay cartridge 200 into the analyzer 100 and press the shown buttons. The analyzer is then capable of performing the immunoassay, acquire the result images and analyze the same in a fully automated manner. Further, a control screen 609 is provided which may for example indicate a charging state of the batteries.

Inside printer housing 611, the analyzer is provided with a printer which preferably is a thermal printer. This printer can be used to print the results of the immunoassay. It was found that by the provision of this printer at the bottom of the analyzer, i.e. beneath the oscillator plate, the optical module 300 and the mechanical module 400 assures a compact and stable construction of the analyzer securing stability of the analyzer in particular during oscillations of the oscillator portion 407.

Fig. 3 shows an example of a lateral flow immunoassay strip 700. In the figure, a control line 703 is shown beside a test line 701 whereby the control line can be used to calibrate the acquired image. For example, the image of the strip taken with the CMOS or CCD sensor can be analyzed searching for the control line to determine the position of the result lines. Further, an absorbing pad 705 including for example antibodies is shown similarly as a pad 707 comprising sample and a pad 709 with remaining sample.

Fig. 4 illustrates examples of an immunoassay as may be visible through the result display section 213 of the immunoassay cartridge 200. The left portion of the figure illustrates a result which originally was in color. The right portion of the figure shows a treated result wherein the color result from the left portion of the figure was converted into grey scale. As can be taken from the figure, by the conversion into grey scale an increase of contrast can be achieved. For example, due to the increased contrast, on the right side of the figure, in result "1", a line 800 "OPI" is clearly visible while to the left side, the same line in result "1" is less clear. Similarly, in result image "2", a line 900 can be seen indicating "PCP" whereby the right portion of Fig. 4 clearly shows that by converting a color figure into a grey scale figure, the result contrast can be enhanced. Thus, in a preferred embodiment, the analyzer is adapted to perform image processing of images acquired by the two-dimensional sensor and the image processing includes a conversion of an image acquired from results displayed in the display section from a color image into a grey scale image.

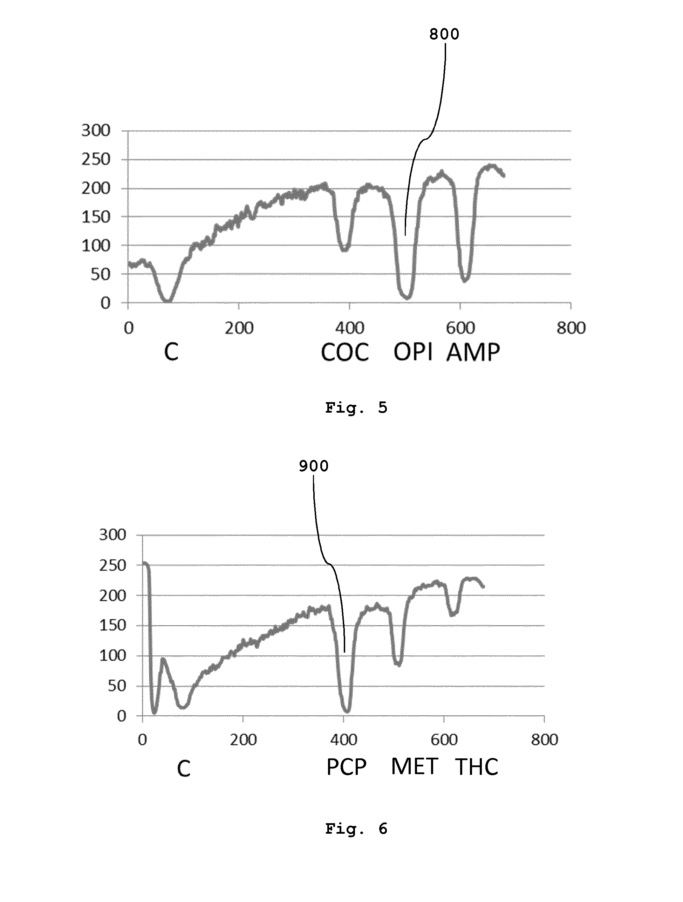

Figs. 5 and 6 illustrate results as shown in Fig. 4 converted into a corresponding curve whereby similar as in Fig. 4, the lines for OPI and PCP have been indicated by reference numerals 800 and 900.

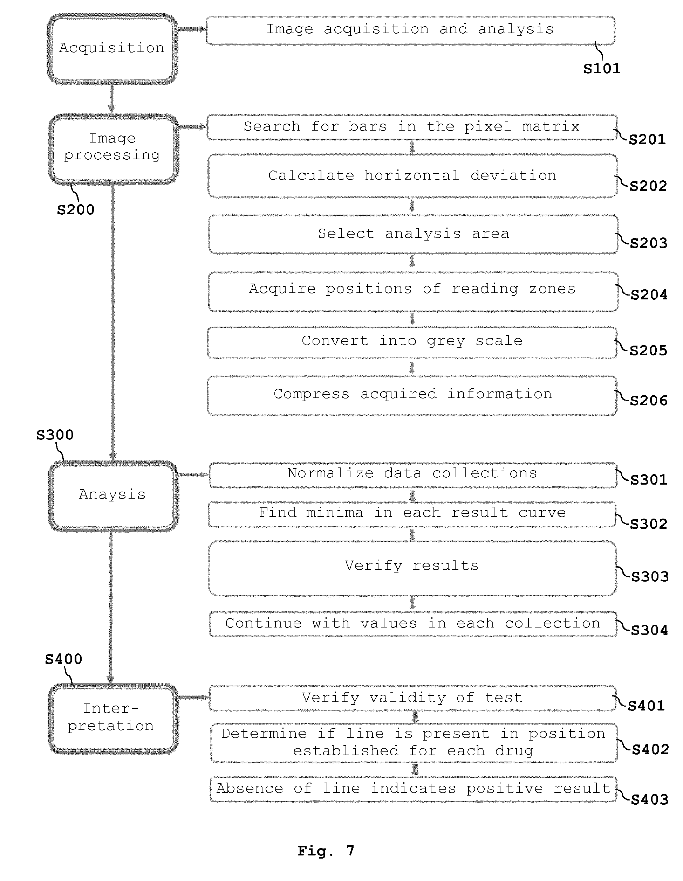

Fig. 7 shows a flowchart illustrating a preferred method of image acquisition and processing and result interpretation. In a preferred embodiment, the analyzer is adapted to perform image processing of images acquired by the two-dimensional sensor and thereby adapted to perform steps, preferably all of the steps, as shown in Fig. 7. As shown, in step S101, an image is taken using the two-dimensional light sensor such as the CMOS sensor 301.

Upon image processing (step S200), bars are searched using a dedicated algorithm within the pixel matrix (step S201). From these bars, an angular deviation with respect to the horizontal orientation is determined (step S202).

Using for example the control line, the analysis area is selected (S203) wherein the result lines indicating the possible presence of drugs is selected. Further, the positions of the reading zones corresponding to the sections of the lateral flow strip are determined (S204).

Then, the results are converted from color scale into grey scale in order to increase the contrast (S205). In order to facilitate the processing, the information is compressed (S206) in order to obtain a data series with values between 0 and 255.

Upon analysis (S300), in order to assure comparability, the data sets are normalized (S301). In each of the data sets, minima are determined (S302) as they are illustrated in the above Figs. 5 and 6. The corresponding minimum values are compared to predetermined threshold values such that the algorithm can automatically interpret the results (S303). Each data set is created in this manner (S304).

Upon interpretation of the results (S400), the validity of test is verified by varying if the control line is detected (S401).

Then, it is determined if lines are present in positions established for each drug (S402).

It is determined if the result is positive by determining the absence of a line (S403). In particular, using the above processing steps, advantageously an automatized acquisition and interpretation of the immunoassay results becomes possible.

## Claims

1. Analyzer (100) adapted to interact with a corresponding immunoassay cartridge (200) for automatically performing an immunoassay and for automatically analyzing and interpreting results of the immunoassay, the analyzer (100) being adapted to at least partially receive the immunoassay cartridge (200);
the analyzer (100) comprising an imaging module (300) including a two-dimensional light sensor (301) at least partially surrounded by a plurality of light sources (303), whereby the light sources (303) and the two-dimensional light sensor (301) are arranged such that when the immunoassay cartridge (200) is at least partially received by the analyzer (100), the light sources (303) are adapted to illuminate at least a result display section (213) and a graphical code (211) provided at the immunoassay cartridge (200), and the two-dimensional light sensor (301) is adapted to acquire images of both the graphical code (211) and the result display section (213);
**characterized in that**
the analyzer (100) further comprises a mechanical module comprising a plurality of actuators and an oscillator portion (407); whereby
each of the actuators is adapted to interact with a corresponding portion of the immunoassay cartridge (200), when the immunoassay cartridge is at least partially received by the analyzer (100) for automatically performing an immunoassay; whereby
the oscillator portion (407) is arranged such that when the immunoassay cartridge (200) is received by the analyzer (100), the oscillator portion (407) is in mechanical contact with the immunoassay cartridge (200), the oscillator portion (407) thereby being adapted to cause the immunoassay cartridge (200) to perform oscillations at a pre-set oscillation frequency; wherein the analyzer (100) further comprises a processing section adapted to process an image of the graphical code (211) acquired by the two-dimensional light sensor (301) to determine the pre-set oscillation frequency; and
the analyzer (100) further comprises a controller connected to the processing section and to the oscillator portion (407), the controller being adapted to receive, from the processing section, the pre-set oscillation frequency, the controller further being adapted to control the oscillator portion (407) to cause the immunoassay cartridge (200) to oscillate at the pre-set oscillation frequency.

2. Analyzer (100) according to claim 1, **characterized in that** the oscillator portion (407) comprises a position detection sensor adapted to detect a position of the immunoassay cartridge (200) with respect to the analyzer, when the immunoassay cartridge (200) is received by the analyzer (100).

3. Analyzer (100) according to any one of claims 1 or 2, **characterized in that** the oscillator portion comprises an oscillator plate (407) and **in that** the analyzer (100) and the immunoassay cartridge (200) are provided such that when the immunoassay cartridge (200) is received by the analyzer (100), the immunoassay cartridge (200) is placed on the oscillator plate (407).

4. Analyzer (100) according to any one of the preceding claims, **characterized in that** the plurality of actuators comprises at least a first actuator (401, 403, 405), a second actuator (401, 403, 405) and a third actuator (401, 403, 405), each being arranged moveable such that when the immunoassay cartridge (200) is received by the analyzer (100), each of the actuators (401, 403, 405) is adapted to be moved from an initial position in a direction towards the immunoassay cartridge (200), whereby the controller is adapted to automatically control movement of each of the actuators, preferably in accordance with information received from the processing section and determined from processing an image of the graphical code (211) acquired by the two-dimensional light sensor (301).

5. Analyzer (100) according to claim 4, **characterized in that** the first actuator (401, 403, 405), the second actuator (401, 403, 405) and the third actuator (401, 403, 405) are linearly moveable in a direction essentially perpendicular to the oscillator plate and **in that** the analyzer (100) comprises at least one actuator position sensor, preferably a potentiometer, for a corresponding one of the actuators (401, 403, 405), which actuator position sensor is connected to the controller and is adapted to convert a position of the corresponding actuator (401, 403, 405) into a signal, preferably a voltage signal.

6. Analyzer (100) according to claim 5, **characterized in that** the controller is adapted to control the position of the corresponding actuator (401, 403, 405) by controlling a corresponding motor which is mechanically connected to the corresponding actuator (401, 403, 405) in response to the voltage signal.

7. Analyzer (100) according to any one of claims 5 to 6, **characterized in that** upon movement from the initial position towards the immunoassay cartridge (200), at least one of the actuators (401, 403, 405) is adapted to compress, preferably to perforate, a corresponding portion of the cartridge (200) upon engagement with the corresponding portion of the immunoassay cartridge (200).

8. Analyzer (100) according to any one of claims 5 to 7, **characterized in that** at least one of the actuators (401, 403, 405) is provided with a guiding portion (415), and **in that** upon movement from the initial position towards the immunoassay cartridge (200), the at least one actuator (401, 403, 405) is adapted to guide a sample collection member at least partially into an inner chamber of the immunoassay cartridge (200).

9. Analyzer (100) according to any one of claims 5 to 8, **characterized in that** at least one of the actuators (401, 403, 405) is provided with a contact portion and **in that** upon movement from the initial position towards the immunoassay cartridge (200) the contact portion of the at least one of the actuators (401, 403, 405) is adapted to come into contact with a corresponding portion of the immunoassay cartridge (200) to move a test strip at least partially into an inner chamber of the immunoassay cartridge (200).

10. Analyzer (100) according to any one of the preceding claims, **characterized in that** the analyzer (100) further comprises a printer, preferably a thermal printer, for automatically printing results of the immunoassay, a screen, preferably an LCD screen, for displaying results of the immunoassay and a storage for storing results of the immunoassay, and incorporates the imaging module (300), the oscillator portion (407), the processing section, the controller, the first actuator (401, 403, 405), the second actuator (401, 403, 405), the third actuator (401, 403, 405), the printer, the screen and the storage in a single portable device with a total weight of less than 10 kg, preferably of less than 7,5 kg, more preferably of less than 5 kg and most preferably of less than 2,5 kg.

11. Method for automatically performing an immunoassay using the analyzer (100) according to any one of claims 1 to 13 and a corresponding immunoassay cartridge (200), the method comprising the following steps, preferably in the given order:
- a step A of inserting a sample collection member comprising a sample to be analyzed by the immunoassay into a first position at least partially inside the immunoassay cartridge (200);
- a step B of inserting the immunoassay cartridge (200) at least partially into the analyzer (100);
- a step D1 of controlling a first actuator (401, 403, 405) to be moved from an initial position towards the immunoassay cartridge (200), thereby compressing a flexible portion of the immunoassay cartridge (200), thus introducing a dilution comprising a substance for analysis of the sample, preferably comprising a buffer for homogenizing reagents, preferably antibodies, into an inner chamber of the cartridge (200);
- a step E of controlling the oscillator portion (407) to cause the immunoassay cartridge (200) to perform first oscillations at a pre-set oscillation frequency;
- a step D2 of controlling a second actuator (401, 403, 405) to be moved from an initial position towards the immunoassay cartridge (200) into engagement with the sample collection member, thereby guiding the sample collection member including the sample to be analyzed at least partially into the inner chamber of the immunoassay cartridge (200) into contact with the dilution to form a mixture of sample and dilution;
- a step F of controlling the oscillator portion (407) to cause the immunoassay cartridge (200) to perform second oscillations at a, preferably the, pre-set oscillation frequency in order to homogenize the mixture of the dilution and the sample;
- a step G of controlling a first actuator (401, 403, 405) to be moved from an initial position towards the immunoassay cartridge (200), thereby moving at least one test strip at least partially into the inner chamber of the immunoassay cartridge (200) into contact with the mixture;
- a step H of waiting for a pre-set time to allow incubation of the test strip with the mixture;
- a step I of acquiring a result image of a result display section (213) of the immunoassay cartridge (200); and
- a step J of processing and analyzing the result image in order to determine as a result result-substances comprised by the sample,
wherein steps D1 to J are performed automatically by the analyzer (100).

12. Method according to claim 11, further comprising
- a step C1 of acquiring an image of a graphical code (211) provided at the immunoassay cartridge (200);
- a step C2 of processing the image of the graphical code (211) in order to determine at least one of the pre-set oscillation frequency and/or the pre-set time, wherein steps C1 and C2 are performed in advance to step D1, and wherein steps C1 to J are performed automatically by the analyzer (100).

13. Method according to claim 12, wherein in step J, it is determined that a result-substance is present in the sample, when a detected signal corresponding to the result-substance is above an internal threshold; and
wherein step C2 further comprises processing the image of the graphical code (211) in order to determine the pre-set internal threshold.

14. Method according to claim 13, wherein in step J, when it is determined that a result-substance is present in the sample, a positive result is output when a concentration of the result-substance in the sample derived from the detected signal is above a threshold concentration; and
wherein step C2 further comprises processing the image of the graphical code (211) in order to determine the threshold concentration.

15. Immunoassay analysis system comprising the analyzer (100) according to any one of claims 1 to 10 and a corresponding immunoassay cartridge (200), preferably wherein the immunoassay cartridge comprises a holder and a cassette for housing an immunoassay strip.

16. Immunoassay analysis system comprising the analyzer (100) according to any one of claims 1 to 10 and a holder for a cassette comprising at least one immunoassay strip, wherein the graphical code is provided on a surface of the holder and wherein the graphical code encodes a pre-set internal threshold and a pre-set threshold concentration; whereby
the analyzer (100) is adapted to automatically analyze a result of an immunoassay and determine that a result-substance is present in a sample when a detected signal corresponding to the result-substance is above the internal threshold; and wherein
the analyzer is further adapted to output a positive result when a concentration of the result-substance in the sample derived from the detected signal is above the threshold concentration.
